## Europäisches Patentamt
## European Patent Office
## Office européen des brevets

(11) Veröffentlichungsnummer: **0 069 260**
**B1**

(12) # EUROPÄISCHE PATENTSCHRIFT

(45) Veröffentlichungstag der Patentschrift:
25.09.85

(21) Anmeldenummer: 82105341.0

(22) Anmeldetag: 18.06.82

(51) Int. Cl.⁴: **A 61 L 17/00,** A 61 K 9/22,
A 61 L 15/03, A 61 L 15/04

(54) **Wirkstoffhaltige Kollageneinlage zum Einführen in Knochen oder Weichteile und Verfahren zu deren Herstellung.**

(30) Priorität: 25.06.81 DE 3124981

(43) Veröffentlichungstag der Anmeldung:
12.01.83 Patentblatt 83/2

(45) Bekanntmachung des Hinweises auf die Patenterteilung:
25.09.85 Patentblatt 85/39

(84) Benannte Vertragsstaaten:
AT BE CH DE FR GB IT LI LU NL SE

(56) Entgegenhaltungen:
DE - A - 2 625 289
DE - A - 2 815 934
GB - A - 1 565 340
GB - A - 2 058 084
US - A - 3 949 073

**Die Akte enthält technische Angaben, die nach dem Eingang der Anmeldung eingereicht wurden und die nicht in dieser Patentschrift enthalten sind.**

(73) Patentinhaber: **Dr. Ruhland Nachf. GmbH, Stadtplatz 7, D-8425 Neustadt/Donau (DE)**

(72) Erfinder: **Steffan, Wolfgang, Am Westhang 2, D-8425 Neustadt/Donau (DE)**
Erfinder: **Stemberger, Axel, Dr.rer.nat., Cramer-Klett-Strasse, D-8014 Neubiberg (DE)**
Erfinder: **Sorg, Karl Heinz, Dr.vet., Regensburger Strasse 4, D-8425 Neustadt/Donau (DE)**

(74) Vertreter: **Lehn, Werner, Dipl.-Ing. et al, Hoffmann, Eitle & Partner Patentanwälte Arabellastrasse 4 (Sternhaus), D-8000 München 81 (DE)**

## Beschreibung

Die Erfindung betrifft eine, im Körper resorbierbare, wirkstoffhaltige Einlage zum Einführen in Knochen oder Weichteile. Sie betrifft insbesondere eine wirkstoffhaltige, blattförmige Kollageneinlage die gegebenenfalls zu einer Stabform aufgewickelt wurde und die nach dem Einbringen in Knochen oder Gewebe dort den Wirkstoff allmählich freigibt.

Es ist bekannt, Kollagen als im Körper bioresorbierbares Material zum Ausfüllen von Knochen- oder Gewebsdeffekten zu verwenden.

Aus der DE-PS 1 293 396 ist die Verwendung von Polyhydroxyessigsäureester zur Herstellung von antibiotikahaltigen resorbierbaren chirurgischen Materialien, z.B. Röhren, bekannt. In der DE-OS 2 051 850 wird eine Kombination aus einem Wirkstoff mit einem Polylaktid oder einem Copolymeren aus Laktid- und Glykolideinheiten beschrieben.

Die vorerwähnten Materialien sind synthetische Polymere oder Copolymere, die im Körper abgebaut werden. Solche Polymere und Copolymere haben zunehmend an Bedeutung gewonnen, weil sie als vollsynthetische Produkte sehr gut reproduzierbar und mit hohem Reinheitsgrad hergestellt werden können. Materialien aus dem vorerwähnten Polymeren und Copolymeren finden auch als synthetische resorbierbare Nahtmaterialien Verwendung.

Kollagen ist ein seit vielen Jahren bekanntes resorbierbares Material und findet Verwendung zum Ausfüllen von Körperhöhlungen, zum Abdekken von Wunden oder auch zum Blutstillen, insbesondere bei und nach chirurgischen Eingriffen. Dabei bestand immer eine gewisse Problematik hinsichtlich des Reinheitsgrades von Kollagen, denn Kollagen ist ein aus Naturprodukten gewonnener Stoff, dessen Reinherstellung schwierig ist. Es gelingt jedoch, Kollagen mit hohem Reinheitsgrad herzustellen, so dass Komplikationen bei der Verwendung von Kollagen für chirurgische Zwecke vermieden werden können.

Aus der DE-OS 2 843 963 sind im Körper resorbierbare geformte Massen auf Basis von Kollagen bekannt. Diese Massen enthalten neben denaturiertem Kollagen ein bioresorbierbares Bindemittel, sowie gegebenenfalls zusätzlich einen Wirkstoff. Als bioresorbierbare Bindemittel kommen insbesondere die vorher erwähnten Polymere und Copolymere der Glykolsäure oder aus Lactid und Glykolid in Frage.

GB-A-2 058 084 betrifft molekular rekonstituierte Kollagenfasern und Kollagenprodukte, wobei in diese Produkte Wirkstoffe eingearbeitet sein können. Die Produkte gemäss GB-A-2 058 084 erhält man, indem man natürliches unlösliches Kollagen mit einer wässrigen Lösung aus Alkalisulfatsalzen und Alkalihydroxid wenigstens 48 Stunden aufschliesst und die Kollagenfasern anquillt, worauf man dann das fettfreie Kollagen mit einer Lösung aus einem Alkalisulfat zur Stabilisierung der interfibularen Bindungen behandelt. Anschliessend wird dann eine wässrige Lösung bei niedrigen Temperaturen ausgefroren und im Vakuum getrocknet.

Aufgabe der Erfindung ist es, eine wirkstoffhaltige Kollageneinlage zum Einführen in Knochen oder Weichteile sowie ein Verfahren zu deren Herstellung zur Verfügung zu stellen, wobei das Verfahren besonders einfach ist.

Diese Aufgabe wird durch eine wirkstoffhaltige Kollageneinlage zum Einführen in Knochen oder Weichteile gelöst, die hergestellt wurde, indem man zu einer Lösung von hochreinem nativen Kollagen, wobei das Kollagen den Faktor Stickstoff zu Hydroxyprolin $\leq$ 3 bis 5 aufweist, eine Lösung oder Suspension des Wirkstoffes zugibt und durch Trocknen mit Warmluft oder Gefriertrocknung ein Blatt in Form einer Faszie herstellt und anschliessend gegebenenfalls zu einem Stab aufwickelt.

Das stabförmige Kollagenprodukt hat ungefähr die Dicke eines Bleistiftes. Es wird zweckmässigerweise hergestellt, indem man einer Kollagenlösung die Wirkstofflösung zugibt und nach Trocknung mit Warmluft oder durch Gefriertrocknung, Kollagen zunächst in Blattform als sogenannte Faszie herstellt. Es kann auch der umgekehrte Weg, je nach der Stabilität des Wirkstoffes, vorgenommen werden, wobei man zunächst die Kollagenfaszie herstellt und dann mit dem Wirkstoff belädt. Dies kann z.B. erfolgen, indem man einen Wirkstoff, z.B. ein Antibiotikum in Salzform, als wässrige Lösung herstellt und auf das Kollagenblatt aufsprüht und dort trocknen lässt. Ebenso ist es möglich, das Antibiotikum oder eine andere pharmakologisch wirksame Verbindung durch Bestreichen, Aufwalzen, durch Tauchen, durch Bestreuen mit einem Wirkstoff in Pulverform oder in ähnlicher Weise zu beladen. Das so mit dem Wirkstoff versehene blattförmige Kollagen kann dann zu einem stabförmigen Produkt von Bleistiftdicke aufgewickelt werden. In dieser Form kann es je nach Dosierung auf beliebige Längen geschnitten und steril bis zur Endanwendung verpackt werden.

Ein besonders geeigneter Wirkstoff in Kombination mit Kollagen ist Gentamycin. Gentamycin, das in Salzform, z.B. als Sulfat, wasserlöslich ist, kann in einem weiten Bereich von etwa 1 bis etwa 100 mg/cm$^2$ Faszie eingearbeitet oder aufgesprüht werden. Das Gentamycinhaltige Kollagenblatt (Kollagenfaszie) wird dann gerollt und getrocknet und behält danach die Stabform bei.

Anstelle oder neben Antibiotika können andere Wirkstoffe, z.B. Sulfonamide, Antiseptika oder gegebenenfalls auch Kortikosteroide eingebracht werden.

Das hier verwendete Kollagen weist eine Reinheit auf, die ausgedrückt wird durch den Faktor Stickstoff: Hydroxyprolin $\leq$ 3. Ein solches Kollagen kann nach der im Beispiel beschriebenen Weise hergestellt werden.

Ein besonderer Vorteil bei dem erfindungsgemässen, mit einem Wirkstoff beladenen stabförmigen Kollagen besteht darin, dass der Wirkstoff sehr gut an dem Kollagen haftet und gegebenenfalls sogar an diesen gebunden wird. In dem Masse wie das Kollagen im Laufe der Zeit abgebaut wird, findet dann die Freigabe des Wirkstoffes statt, so dass eine Retard-Wirkung vorliegt. Ein weiterer Vorteil ist darin zu sehen, dass die Trägermatrix Kollagen als Leitschiene für einsprossende Fibroblasten dient und so in bekannter Weise die Wundheilung fördert.

Beispiel

Das nachfolgend verwendete hochreine, native Kollagen wird wie folgt hergestellt:

Von allen Pigmentschichten und Muskelresten befreite frische Rindersehnen wurden homogenisiert und eine Menge, die 100g Trockengewicht entspricht, in 3 l 0,05m Zitratpuffer (pH 3,7) 24 Stunden lang extrahiert und anschliessend gegen 1% Essigsäure 12 Stunden lang dialysiert. Das in 3 l 1%-iger Essigsäure suspendierte Gewebe wurde 48 Stunden bei 15°C unter ständigem Rühren mit Pepsin im Verhältnis Kollagen zu Pepsin gleich 50:1 inkubiert.

Der Ansatz wurde mit 1%-iger Essigsäure auf 5 l verdünnt und durch Zentrifugation von den ungelösten Sehnenfragmenten befreit.

Die viskose Kollagenlösung wird gegen alkalisiertes Leitungswasser (pH 8,0) dialysiert und dann scharf zentrifugiert. Der Rückstand wird erneut in 5 l 1%-iger Essigsäure gelöst und dialysiert. Dieser Vorgang wird wiederholt, bis der Faktor Stickstoff: Hydroxyprolin ≦ ist. Nach dem letzten Dialysieren wird mittels 0,05% Essigsäure eine 1,5%-ige Kollagenlösung hergestellt.

Herstellung der wirkstoffhaltigen Kollageneinlage

10 ml der wie vorstehend erhaltenen 1,5%-igen Kollagenlösung wurden in eine Schale von 10 × 10 cm gegeben. Dazu wurden 100 mg Gentamycin gegeben. In der Schale wurde dann die Gefriertrocknung der Lösung vorgenommen, wobei man einen Schwamm, der die Umrisse der Schale hatte, erhielt. Dieser Schwamm wurde dann zur Faszie verpresst und gegebenenfalls zu einem stabförmigen Produkt von Bleistiftdicke aufgewickelt.

Die so erhaltene Gentamycin enthaltende Kollageneinlage wird steril verpackt und mit Ethylenoxid in an sich bekannter Weise sterilisiert.

Anwendung

Wird die vorher beschriebene, 100 mg Gentamycin enthaltende Kollageneinlage in die Tibia eingeführt, so wird sie dort innerhalb von 3 Wochen vollständig resorbiert. Während dieser Zeit wird Gentamycin freigegeben.

**Patentansprüche**

1. Wirkstoffhaltige Kollageneinlage zum Einführen in Knochen oder Weichteile, dadurch gekennzeichnet, dass sie hergestellt wurde, indem man zu einer Lösung von hochreinem nativen Kollagen, wobei das Kollagen den Faktor Stickstoff zu Hydroxyprolin ≦ 3 bis 5 aufweist, eine Lösung oder Suspension des Wirkstoffes zugibt und durch Trocknen mit Warmluft oder Gefriertrocknung ein Blatt in Form einer Faszie herstellt und anschliessend gegebenenfalls zu einem Stab aufwickelt.

2. Wirkstoffhaltige Kollageneinlage gemäss Anspruch 1, dadurch gekennzeichnet, dass der Wirkstoff ein Antibiotikum, vorzugsweise Gentamycin ist.

3. Verfahren zur Herstellung einer wirkstoffhaltigen Kollageneinlage gemäss Anspruch 1, dadurch gekennzeichnet, dass man hochreines, natives Kollagen mit dem Faktor Stickstoff: Hydroxyprolin ≦ 3 bis 5 mit einem Wirkstoff belädt und einem Blatt verarbeitet und gegebenenfalls dann einem Stab aufwickelt.

**Claims**

1. Collagen insert containing an active ingredient for introduction into bones or soft parts, characterized in that it is prepared by adding to a solution of high-purity native collagen, wherein the collagen has a factor of nitrogen to hydroxyproline ≦ 3 to 5, a solution or suspension of said active ingredient and then by drying with hot air or by freeze-drying a sheet is produced in the form of a fascia and finally it is optionally wound to form a rod.

2. Collagen insert containing an active ingredient as in claim 1, characterized in that said active ingredient is an antibiotic, preferably gentamycin.

3. Process for the preparation of a collagen insert containing an active ingredient as in claim 1, characterized in that a high-purity native collagen having the factor nitrogen: hydroxyproline ≦ 3 to 5 is charged with an active ingredient and is processed into a sheet and then is optionally wound into a rod.

**Revendications**

1. Inclusion de collagène contenant une substance active pour introduction dans des os ou des tissus mous, caractérisée en ce qu'elle a été préparée en ajoutant à une solution de collagène natif de grande pureté où le collagène présente un rapport de l'azote à l'hydroxyproline ≦ 3 à 5 une solution ou une suspension de la substance active et en préparant par séchage à l'air chaud ou par cryodessication une feuille sous forme fasciculée qui est ensuite éventuellement enroulée en une baguette.

2. Inclusion de collagène contenant une substance active selon la revendication 1, caractérisée en ce que la substance active est un antibiotique, de préférence la gentamycine.

3. Procédé de préparation d'une inclusion de collagène contenant une substance active selon la revendication 1 caractérisé en ce qu'on charge avec une substance active du collagène natif de grande pureté ayant un rapport azote: hydroxyproline ≦ 3 à 5, puis on la transforme en une feuille et éventuellement on l'enroule en une baguette.